# EUROPÄISCHE PATENTSCHRIFT

(11) **EP 3 082 415 B1**
(45) Veröffentlichungstag und Bekanntmachung des Hinweises auf die Patenterteilung: **08.05.2019**
(21) Anmeldenummer: 14821624.5
(22) Anmeldetag: 19.12.2014
(51) Int. Cl.: A01N 25/08, A01N 59/16, C08K 3/22, C08K 3/36, C08K 5/00, C09D 1/00, C09D 5/14, C09D 7/63

(54) **ANTIMIKROBIELL WIRKSAMER VERBUNDWERKSTOFF UND VERFAHREN ZUM HERSTELLEN DESSELBEN**
ANTIMICROBIAL COMPOSITE MATERIAL AND PROCESS FOR THE PREPARATION THEREOF
MATÉRIAU COMPOSITE ANTIMICROBIEN ET PROCESSUS DE PRÉPARATION

(30) Priorität: 19.12.2013 DE 102013114575
(43) Veröffentlichungstag der Anmeldung: 26.10.2016
(73) Patentinhaber: Amistec GmbH & Co. KG, 6345 Kössen (AT)
(72) Erfinder: GUGGENBICHLER, Josef-Peter, A-6345 Kössen (AT)
(74) Vertreter: Heubeck, Christian
(86) Internationale Anmeldenummer: PCT/EP2014/078821
(87) Internationale Veröffentlichungsnummer: WO 2015/091993

(56) Entgegenhaltungen:
- EP-A1- 0 956 770
- EP-A1- 2 255 878
- WO-A1-2013/153124
- WO-A1-2014/174084
- WO-A2-2007/079210
- WO-A2-2008/058707
- WO-A2-2010/136792
- DE-A1-102011 085 862
- US-A1- 2012 201 861
- TETSU TATSUMA ET AL: "Bactericidal effect of an energy storage TiO2-WO3 photocatalyst in dark", ELECTROCHEMISTRY COMMUNICATIONS, ELSEVIER, AMSTERDAM, NL, Bd. 5, Nr. 9, 1. September 2003 (2003-09-01), Seiten 793-796, XP002656981, ISSN: 1388-2481, DOI: 10.1016/J.ELECOM.2003.07.003 [gefunden am 2003-08-23]
- KATHRIN LORENZ ET AL: "Anodic TiO2 nanotube layers electrochemically filled with MoO3 and their antimicrobial properties", BIOINTERPHASES, Bd. 6, Nr. 1, 17. März 2011 (2011-03-17), Seiten 16-21, XP55169086, ISSN: 1934-8630, DOI: 10.1116/1.3566544
- CORDT ZOLLFRANK ET AL: "Antimicrobial activity of transition metal acid MoOprevents microbial growth on material surfaces", MATERIALS SCIENCE AND ENGINEERING C, ELSEVIER SCIENCE S.A, CH, Bd. 32, Nr. 1, 22. September 2011 (2011-09-22), Seiten 47-54, XP028112650, ISSN: 0928-4931, DOI: 10.1016/J.MSEC.2011.09.010 [gefunden am 2011-09-29]
- Nathalie Tétault ET AL: "Biocidal activity of metalloacid-coated surfaces against multidrug-resistant microorganisms", Antimicrobial Resistance & Infection Control, 14. November 2012 (2012-11-14), Seiten 1-6, XP55168973, DOI: 10.1186/2047-2994-1-35 Gefunden im Internet: URL:http://www.aricjournal.com/content/pdf /2047-2994-1-35.pdf [gefunden am 2015-02-11]
- S Shafaei ET AL: "Molybdenum and tungsten oxides as innovative antimicrobial materials", Book of Abstracts, 21. November 2012 (2012-11-21), Seiten 314-314, XP55168956, Lisbon, Portugal Gefunden im Internet: URL:http://www.formatex.info/icar2012/abst racts/htm/223.pdf [gefunden am 2015-02-11]
- MAXIMILIAN LACKNER ET AL: "Polymorphs of molybdenum trioxide as innovative antimicrobial materials", SURFACE INNOVATIONS, Bd. 1, Nr. 4, 5. Oktober 2013 (2013-10-05) , Seiten 202-208, XP55168959, ISSN: 2050-6252, DOI: 10.1680/si.13.00021
- DATABASE WPI Week 199704 Thomson Scientific, London, GB; AN 1997-038305 XP002735821, -& JP H08 296031 A (TOPPAN PRINTING CO LTD) 12. November 1996 (1996-11-12)
- Hu Tang ET AL: "Highly antibacterial materials constructed from silver molybdate nanoparticles immobilized in chitin matrix", CHEMICAL ENGINEERING JOURNAL, vol. 234, 5 September 2013 (2013-09-05), pages 124-131, XP55393557, CH ISSN: 1385-8947, DOI: 10.1016/j.cej.2013.08.096
- Qianping Wang ET AL: "Method for preparing three-component coprecipitation molybdate antimicrobial by using microwave heating" In: "EPODOC Database", 24 September 2008 (2008-09-24), XP55393560, pages 1-2, & CN 101 268 784 A (UNIV HEBEI POLYTECHNIC [CN]) 24 September 2008 (2008-09-24)
- DATABASE WPI Week 201122 Thomson Scientific, London, GB; AN 2011-A22497 XP55447126, -& CN 101 891 252 A (UNIV HEBEI UNITED) 24 November 2010 (2010-11-24)
- DATABASE WPI Week 201144 Thomson Scientific, London, GB; AN 2011-E30870 XP55447138, -& CN 101 983 930 A (UNIV HEBEI UNITED) 9 March 2011 (2011-03-09)
- Yan Yan Meng ET AL: "Preparation of Molybdates with Antibacterial Property", Key Engineering Materials, vol. 368-372, 1 January 2008 (2008-01-01), pages 1516-1518, XP55256025, Aedermannsdorf ISSN: 0252-1059, DOI: 10.4028/www.scientific.net/KEM.368-372.151 6

## Beschreibung

Die Erfindung betrifft ein Verfahren zum Herstellen eines antimikrobiell wirksamen Verbundwerkstoffs, einen antimikrobiell wirksamen Verbundwerkstoff sowie die Verwendung eines derartigen antimikrobiell wirksamen Verbundwerkstoffs.

Oberflächen von Gegenständen lassen sich nach unterschiedlichen Verfahren antimikrobiell ausstatten. Neben organischen Bioziden werden häufig Metalle und Metallverbindungen zu diesem Zweck eingesetzt und zur antimikrobiellen Ausstattung unterschiedlicher Verbundmaterialien eingesetzt. Nach der so genannten oligodynamischen Reihe ist eine Vielzahl an Metallionen antimikrobiell wirksam, darunter beispielsweise Ag⁺, Cd²⁺, Hg²⁺ und Cu²⁺. Silber wird neben Kupfer besonders häufig eingesetzt, wobei mehrere prinzipielle Möglichkeiten bekannt sind. Bei der ersten Möglichkeit wird das elementare Metall mit einer großen Oberfläche bereitgestellt, so dass sich die entsprechenden Metallionen an der Oberfläche bilden können. Häufig werden daher Nanopartikel, geschäumtes Metall oder auf einem Träger fixierte Nanopartikel verwendet. Die zweite Möglichkeit umfasst die Bereitstellung von löslichen Metallsalzen, die beispielsweise in Zeolithen oder direkt im Verbundmaterial eingebracht sind. Weiterhin ist es möglich, antimikrobiell wirksame Metallionen über die elektrochemische Spannungsreihe darzustellen. Hierzu können vergleichsweise unedlere Metalle wie Silber oder Kupfer galvanisch mit einem edleren Metall wie beispielsweise Platin gekoppelt werden.

Als nachteilig an organischen Bioziden ist jedoch der Umstand anzusehen, dass diese als aktive Wirkstoffe aus dem Verbundwerkstoff herausgelöst und in den Bakterien-Metabolismus eingebaut werden. Zusätzlich besteht bei aktiven Bioziden eine hohe Tendenz zur Entwicklung von Resistenzen und Kreuzresistenzen. Die genannten Edelmetalle bzw. Edelmetallionen sind einerseits vergleichsweise teuer und werden andererseits durch schwefelhaltige Verbindungen sowie durch hohe Elektrolytkonzentrationen nahezu völlig inaktiviert.

Die internationale Patentanmeldung WO 2008/058707 A2 beschreibt einen antimikrobiellen Verbundwerkstoff, welcher wenigstens eine Molybdän- und/oder Wolfram-haltige anorganische Verbindung, z.B. MoO₂, MoO₃ oder WO₃ enthält. Verbundwerkstoffe mit Molybdaten der Summenformel Aⁿ⁺_{z} Mo₄ werden nicht beschrieben.

WO 2013/153124 A1 beschreibt ein Verbundmaterial, welches ein Hydrophilierungsmittel, ein Trägermaterial und eine antimikrobiell wirksame Metallverbindung enthält. Die antimikrobiell wirksame Metallverbindung ist ein Übergangsmetalloxid, insbesondere MoO₃ oder WO₃. Verbundwerkstoffe, welche eine Verbindung der Summenformel Aⁿ⁺_{z} MoO₄ enthalten, werden nicht beschrieben.

Die chinesische Patentanmeldung CN 101891252 A offenbart die Herstellung von Zinkmolybdat aus Natriumorthomolybdat und die Verwendung des Produkts in Form von Partikeln mit einer Teilchengröße von 2 µm zur Herstellung antimikrobieller Verbundmaterialien. Kleinere Partikel mit einer mittleren Korngröße von 1,0 µm und weniger werden nicht beschrieben.

Yanyan Meng et al., Key Engineering Materials, Band 368-372, 1. Januar 2008, Seiten 1516-1518, offenbart die Verwendung von 3 Gew.-% Zinkmolybdat mit einer Partikelgröße von 0,1 µm zur Herstellung eines antimikrobiell wirksamen Verbundwerkstoffes, namentlich eines Anstrichmittels. Es wird jedoch kein Verbundwerkstoff beschrieben, der zusätzlich zum Molybdat noch ein Hydrophilierungs- und/oder Hydroskopierungsmittel ausgewählt aus Kieselgel und pyrogenem Siliziumdioxid enthält.

Aufgabe der vorliegenden Erfindung ist es, ein Verfahren zum Herstellen eines antimikrobiell wirksamen Verbundwerkstoffs anzugeben, welcher kostengünstiger ist und eine verbesserte Wirksamkeit besitzt. Weitere Aufgaben der Erfindung bestehen darin, einen entsprechenden antimikrobiell wirksamen Verbundwerkstoff sowie eine Verwendung eines solchen Verbundwerkstoffs anzugeben.

Die Aufgaben werden erfindungsgemäß durch ein Verfahren mit den Merkmalen des Patentanspruchs 1, einen antimikrobiell wirksamen Verbundwerkstoff mit den Merkmalen des Patentanspruchs 10 sowie durch die in Anspruch 11 angegebene Verwendung eines derartigen Verbundwerkstoffs gelöst. Vorteilhafte Ausgestaltungen mit zweckmäßigen Weiterbildungen der Erfindung sind in den Unteransprüchen angegeben.

Ein erster Aspekt der Erfindung betrifft ein Verfahren zum Herstellen eines antimikrobiell wirksamen Verbundwerkstoffs wie in Anspruch 1 definiert, bei welchem wenigstens eine Molybdän-haltige anorganische Verbindung mit wenigstens einem weiteren Werkstoff verbunden wird. Unter einem Verbundwerkstoff wird im Rahmen der vorliegenden Erfindung ein Werkstoff verstanden, der aus zwei oder mehr miteinander verbundenen Materialien besteht, wobei zumindest eines der Materialien die wenigstens eine Molybdän-haltige anorganische Verbindung ist. Weitere Materialien des erfindungsgemäßen Verbundwerkstoffs können dabei selbst wieder Verbundwerkstoffe sein. Da Molybdän-haltige Verbindungen im Vergleich beispielsweise zu Nanosilber, Kupfer, organischen Bioziden und dergleichen wesentlich kostengünstiger sind und bereits in geringen Mengen eine starke antimikrobielle Wirkung zeigen, kann der erfindungsgemäß hergestellte Verbundwerkstoff somit kostengünstiger hergestellt werden und besitzt eine verbesserte Wirksamkeit. Darüber hinaus werden Molybdän-haltige Verbindungen durch schwefelhaltige Verbindungen oder durch hohe Elektrolytkonzentrationen nicht inaktiviert, sondern behalten ihre Wirksamkeit. Obwohl der genaue Mechanismus der antimikrobiellen Wirksamkeit noch nicht abschließend geklärt ist, geht die Anmelderin davon aus, dass die Hauptwirkung dadurch erzielt wird, dass sich Molybdän-haltige anorganische Verbindungen bei Kontakt mit Wasser, Luftfeuchtigkeit etc. in Molybdänsäure, Oligomolybdate und ähnliche sauren Molybdän-Verbindungen umwandeln, welche zu einer Absenkung des pH-Werts führen. Aufgrund dieses sauren pH-Werts ist die Haftfähigkeit pathogener Keime von vornherein stark reduziert. Neben einer verminderten Adhärenz von Keimen werden aber auch eine verminderte Festigkeit der Adhärenz, eine Hemmung der Proliferation, eine Hemmung der Biofilmbildung und eine antimikrobielle Wirksamkeit bei bereits erfolgter Biofilmbildung erreicht. Dies ist beispielsweise in Krankenhäusern, Pflegeheimen usw. von besonderer Bedeutung, da Mikroorganismen im Biofilm durch Antibiotika, organische Biozide, Desinfektionsmittel und dergleichen nicht oder zumindest nicht dauerhaft entfernbar sind. Da eine Absenkung des pH-Werts lediglich im Bereich der Oberflächengrenzschicht des Verbundwerkstoffs bzw. eines aus diesem gefertigten Bauteils oder Produkts benötigt wird, sind entsprechend geringe Mengen an Molybdän-haltigen Verbindungen im Bereich der Oberfläche ausreichend. Nachdem Molybdän-haltige anorganische Verbindungen generell zumindest unter normalen Umweltbedingungen schlecht bis kaum wasserlöslich sind, unterbleibt zudem ein Auswaschen oder Abbau der anorganischen Verbindungen, so dass die antimikrobielle Wirksamkeit im Unterschied zum Stand der Technik über die gesamte Lebensdauer des Verbundwerkstoffs aufrechterhalten wird. Demgegenüber werden aktive Biozide mit der Zeit aus Verbundwerkstoffen bzw. aus deren Oberflächen herausgelöst und in den Bakterien-Metabolismus eingebaut. Daher besteht bei aktiven Bioziden eine hohe Tendenz zur Entwicklung von Resistenzen und Kreuzresistenzen. Demgegenüber wirkt der antimikrobiell ausgestattete Verbundwerkstoff als passives Biozidprodukt, wodurch neben einer Langzeitwirksamkeit auch eine fehlende Resistenzentwicklung bzw. Resistenzinduktion sichergestellt werden. Wie weiterhin erkannt wurde, sind Molybdän-haltige Verbindungen für Menschen und Tiere nicht toxisch und weisen damit eine exzellente Biokompatibilität auf. Der wenigstens eine weitere Werkstoff kann grundsätzlich aus einer beliebigen Werkstoffklasse stammen und ein anorganischer, metallischer, keramischer und/oder organischer Werkstoff sein. Bevorzugt umfasst der erfindungsgemäß hergestellte Verbundwerkstoff als weiteren Werkstoff zumindest ein organisches Polymer bzw. ein Compound und/oder ein Silikon. Der Verbundwerkstoff kann mit Hilfe des erfindungsgemäßen Verfahrens grundsätzlich als Schicht-Verbund, Faser-Verbund, Teilchen-Verbund oder Durchdringungsverbund ausgebildet sein. Der erfindungsgemäß hergestellte Verbundwerkstoff kann bei Standardbedingungen fest oder flüssig sein. Beispielsweise kann der Verbundwerkstoff in Form einer Lösung, Suspension und/oder Dispersion, beispielsweise als Lack bzw. flüssiges Beschichtungsmittel hergestellt werden. Grundsätzlich kann vorgesehen sein, dass die wenigstens eine Molybdän-haltige Verbindung zumindest im Wesentlichen ausschließlich im Bereich der Oberfläche des Verbundwerkstoffs angeordnet wird, da hier die antimikrobielle Wirkung zu erzielen ist. Beispielsweise kann die Molybdän-haltige Verbindung als Schicht oder Bestandteil einer Schicht auf ein Substrat bzw. ein Trägermaterial des Verbundwerkstoffs aufgebracht werden. Grundsätzlich können dabei nur ein oder mehrere Bereiche der Oberfläche oder die gesamte Oberfläche des Verbundwerkstoffs antimikrobiell ausgestattet werden. Alternativ oder zusätzlich kann die wenigstens eine Molybdän-haltige Verbindung auch innerhalb des Verbundwerkstoffs angeordnet werden bzw. im Verbundwerkstoff verteilt vorliegen. Hierdurch kann die antimikrobielle Wirkung auch bei einer oberflächlichen Abnutzung des Verbundwerkstoffs dauerhaft aufrechterhalten werden. In Abhängigkeit des Verwendungszwecks kann der Verbundwerkstoff im Rahmen der vorliegenden Erfindung grundsätzlich als Halbzeug, das heißt als "halbfertiges Material" ausgebildet werden, das erst nach weiteren Verarbeitungsschritten zu seinem endgültigen Zweck verwendet wird. Alternativ kann der Verbundwerkstoff bereits als fertiges Bauteil ausgebildet werden, welches ohne weitere Verarbeitungsschritte zu seinem jeweiligen Zweck verwendet werden kann. Der erfindungsgemäß hergestellte Verbundwerkstoff kann darüber hinaus grundsätzlich frei von elementarem Molybdän, MoO₂, MoO₃, Molybdänkarbid, Molybdännitrid, Molybdänsilizid oder Molybdänsulfid, Molybdänhexacarbonyl, Molybdänacetylacetonat und/oder Molybdän-haltigen Legierungen ausgebildet sein. Insbesondere ist das wenigstens eine weitere Material kein Molybdän bzw. keine Molybdän -Legierung. Ebenso kann der Verbundwerkstoff frei von nicht-säurebildenden Metalloxiden wie Zinkoxid, Titanoxid, Titandioxid, Aluminiumoxid oder sonstigen nicht-säurebildenden Photokatalysatoren ausgebildet sein. Weiterhin kann das erfindungsgemäße Verfahren im Unterschied zum Stand der Technik grundsätzlich ohne die Verwendung zusätzlicher antimikrobiell wirksamer Verbindungen wie beispielsweise Silber, insbesondere Nanosilber, bzw. Silberverbindungen, insbesondere löslicher Silberverbindungen wie Silbernitrat und dergleichen, Kupfer, organische Biozide, Zeolithe oder dergleichen durchgeführt werden, wodurch neben einer besseren Umweltverträglichkeit des erfindungsgemäß hergestellten Verbundwerkstoffs auch erhebliche Kostensenkungen gegeben sind.

In einer vorteilhaften Ausgestaltung der Erfindung ist vorgesehen, dass wenigstens eine Molybdän-haltige anorganische Verbindung verwendet wird, welche dotiert und/oder undotiert und/oder kristallwasserfrei und/oder kristallwasserhaltig ist. Unter einer Dotierung wird im Rahmen der vorliegenden Erfindung das Einbringen von Fremdatomen in die Molybdän-haltige Verbindung verstanden, wodurch eine optimale Anpassbarkeit der antimikrobiellen Wirkung an unterschiedliche Einsatzzwecke und Anforderungsprofile ermöglicht ist. Die Menge an Fremdatomen beträgt dabei grundsätzlich zwischen etwa 0,1 und 1000 ppm, vorzugsweise zwischen 100 ppm und 600 ppm, insbesondere zwischen 300 ppm und 550 ppm. Alternativ oder zusätzlich können sowohl kristallwasserfreie Molybdän-Verbindungen als auch Hydrate, das heißt je nach Verbindung Monohydrate, Dihydrate, Trihydrate usw. der anorganischen Molybdän-Verbindungen, einzeln oder in beliebiger Kombination verwendet werden. Ebenso kann die wenigstens eine anorganische Molybdän-Verbindungen in einer beliebigen Kristallkonfigurationen bzw. Kristallgitterstruktur, als Mischkristall und/oder amorph vorliegen.

In einer weiteren vorteilhaften Ausgestaltung der Erfindung ist vorgesehen, dass als weitere anorganische Verbindung MO₃₋ₓ mit M=Mo und/oder W und 0<x<1 verwendet wird. Mit anderen Worten ist es vorgesehen, dass als weitere anorganische Verbindung ein gegenüber MoO₃ und/oder WO₃ sauerstoffdefizitäres Oxid verwendet wird, dessen Sauerstoffgehalt zwischen denjenigen von MoO₃/WO₃ und MoO₂/WO₂ liegt. Dementsprechend kann x beispielsweise Werte von 0,01, 0,02, 0,03, 0,04, 0,05, 0,06, 0,07, 0,08, 0,09, 0,10, 0,11, 0,12, 0,13, 0,14, 0,15, 0,16, 0,17, 0,18, 0,19, 0,20, 0,21, 0,22, 0,23, 0,24, 0,25, 0,26, 0,27, 0,28, 0,29, 0,30, 0,31, 0,32, 0,33, 0,34, 0,35, 0,36, 0,37, 0,38, 0,39, 0,40, 0,41, 0,42, 0,43, 0,44, 0,45, 0,46, 0,47, 0,48, 0,49, 0,50, 0,51, 0,52, 0,53, 0,54, 0,55, 0,56, 0,57, 0,58, 0,59, 0,60, 0,61, 0,62, 0,63, 0,64, 0,65, 0,66, 0,67, 0,68, 0,69, 0,70, 0,71, 0,72, 0,73, 0,74, 0,75, 0,76, 0,77, 0,78, 0,79, 0,80, 0,81, 0,82, 0,83, 0,84, 0,85, 0,86, 0,87, 0,88, 0,89, 0,90, 0,91, 0,92, 0,93, 0,94, 0,95, 0,96, 0,97, 0,98 oder 0,99 sowie sämtliche möglichen Zwischenwerte annehmen. Mit Hilfe dieser Verbindungen, einzeln oder in beliebiger Kombination, werden auf besonders einfache und kostengünstige Weise ein möglichst niedriger Oberflächen-pH-Wert und damit eine besonders gute antimikrobielle Wirksamkeit erzielt, da sich alle genannten Verbindungen bei Kontakt mit Wasser in Molybdän- bzw. Wolframsäure und/oder höhere saure Oligomolybdate bzw. Oligowolframate umwandeln. Darüber hinaus weisen die genannten Verbindungen aufgrund der unterschiedlichen Oxidationsstufen von Mo/W, das heißt im Wesentlichen +IV, +V und/oder +VI, ein vergleichsweise hohes Oxidationspotential, magnetische Eigenschaften und/oder elektrische Leitfähigkeit auf, wodurch zusätzliche antimikrobielle Effekte erzielt werden können.

Besonders einfach und kostengünstig können die zusätzlichen Verbindungen der allgemeinen Formel MO₃₋ₓ durch partielle Oxidation von M und/oder MO₂ und/oder durch partielle Reduktion von MO₃ hergestellt werden. Mit anderen Worten können die Metalle Mo/W und/oder die Dioxide MoO₂/WO₂ aufoxidiert werden, um zu den Verbindungen der allgemeinen Formel MO₃₋ₓ zu gelangen. Alternativ oder zusätzlich können die jeweiligen Trioxide MoO₃ und/oder WO₃ als Edukte verwendet und partiell reduziert werden.

In einer weiteren vorteilhaften Ausgestaltung der Erfindung ist vorgesehen, dass die zusätzliche Verbindung MO₃₋ₓ mittels eines Wirbelschichtreaktors und/oder durch chemische Gasphasenabscheidung und/oder durch physikalische Gasphasenabscheidung und/oder durch Sputtern und/oder plasmaassistiert und/oder in einer kontrollierten Atmosphäre hergestellt wird. Die Verwendung eines Wirbelschichtreaktors bietet verschiedene Vorteile. Einerseits können die vorzugsweise partikelförmigen Edukte kontrolliert mit einem Fluid in einen fluidisierten Zustand versetzt werden, wobei als Fluid beispielsweise ein Reaktionsgas oder -gasgemisch verwendet werden kann, mittels welchem die partielle Oxidation und/oder Reduktion durchführbar ist. Alternativ oder zusätzlich kann über das Fluid eine gewünschte Reaktionstemperatur eingestellt werden. Ebenso können die fluidisierten Edukte durch eine entsprechende Fluidströmung, beispielsweise ringförmig, an einer Energiequelle, beispielsweise einer Flamme und/oder einer Plasmaquelle, vorbeigeführt werden, wodurch die Kontaktzeit für die einzelnen Partikel und damit ihr Oxidations- bzw. Reduktionsgrad besonders präzise eingestellt werden können. Ebenso kann der Eduktstrom gezielt mit einem Reaktand bzw. Reaktandengemisch versetzt oder an einem Reaktandenstrom vorbeigeleitet werden. Weiterhin können mit Hilfe des Wirbelschichtreaktors zusätzlich zu der Oxidation bzw. Reduktion weitere Funktionalisierungen der anorganischen Molybdän- und/oder Wolfram-Verbindungen vorgenommen werden. Beispielsweise können die Molybdän- und/oder Wolfram-Verbindungen vor, während und/oder nach der Oxidation bzw. Reduktion mit einer Funktionsschicht, beispielsweise einer Reaktandenschicht, einer Hydrophilierungsschicht und Ähnlichem versehen werden. Mit Hilfe von chemischer und/oder physikalischer Gasphasenabscheidung kann der erfindungsgemäße Verbundwerkstoff vorteilhafterweise unmittelbar hergestellt werden, indem der wenigstens eine weitere Werkstoff direkt mit der Molybdän- und/oder Wolfram-haltigen Verbindung beschichtet wird. Entsprechende Vorteile ergeben sich bei einem Schichtauftrag mittels Sputtern. Weiterhin kann vorgesehen sein, dass die Reaktion durch Einstellen einer kontrollierten Atmosphäre gesteuert wird. Beispielsweise kann eine Reduktion in einer kontrollierten Wasserstoffatmosphäre stattfinden, während zu Einstellung oxidativer Bedingungen beispielsweise Sauerstoff, Ozon, Wasserstoffperoxid, Chlor oder andere oxidative Verbindungen, einzeln und in beliebiger Kombination verwendet werden können. Grundsätzlich ist jedoch auch eine nasschemische Herstellung möglich.

In der Erfindung ist vorgesehen, dass als anorganische Verbindung wenigstens eine Verbindung der Summenformel Aⁿ⁺_{z}MO₄, in welcher M Mo bedeutet, A wenigstens ein von Mo verschiedenes Metall-Ion aus einer Gruppe, die aus Na, K, Mg, Ca, Ag, Cu, Bi, V, Ti und Zn besteht und/oder NH₄⁺ bezeichnet und n*z=+2 beträgt, verwendet wird. Durch die Verwendung einer oder mehrerer der genannten Verbindungen wird überraschenderweise neben einer guten antimikrobiellen Wirksamkeit auch eine besonders hohe Lichtstabilität, insbesondere gegenüber UV-Licht, erzielt. Somit wird das Auftreten unerwünschter Verfärbungen an der Oberfläche des erfindungsgemäß hergestellten Verbundwerkstoffs bzw. eines daraus hergestellten Bauteils besonders zuverlässig verhindert. Darüber hinaus weisen derartige Molybdate eine besonders geringe Wasserlöslichkeit auf und sind zumindest im Wesentlichen farblos bzw. weiß. Hierdurch eignet sich der erfindungsgemäß hergestellte Verbundwerkstoff besonders gut zur Herstellung von Gegenständen, für die eine neutrale, weiße Oberfläche gewünscht ist. Umgekehrt kann aufgrund der neutral-weißen Farbe der Oberfläche aber auch eine einfache Einfärbung durch Zusatz entsprechender Farbstoffe bzw. Farbpigmente vorgenommen werden.

Indem A der Summenformel Aⁿ⁺_{z}MO₄ ausgewählt wird aus einer Gruppe, die Na, K, Mg, Ca, Ag, Cu, Bi, V, Ti und Zn umfasst, können die Löslichkeit, die Farbe und die antimikrobielle Wirksamkeit des Verbundwerkstoffs optimal an seinen jeweiligen Einsatzzweck angepasst werden. Mit Hilfe der genannten Verbindungen, einzeln und in beliebiger Kombination, kann zudem die Adhäsion von Mikroorganismen an der Oberfläche des Verbundwerkstoffs zusätzlich erschwert werden. Dies verhindert besonders effektiv die Besiedelung der Oberfläche des Verbundwerkstoffs. Die genannten Molybdate können besonders schnell, einfach und kostengünstig durch gemeinsames Erhitzen der gewünschten Carbonate, beispielsweise Na₂CO₃, ZnCO₃, CaCO₃ usw., mit MoO₃ hergestellt werden.

In einer weiteren vorteilhaften Ausgestaltung der Erfindung ist vorgesehen, dass als weitere anorganische Verbindung eine Molybdän- und Wolfram-haltige anorganische Mischverbindung verwendet wird. Aufgrund der ähnlichen Atomradien von Molybdän und Wolfram können beide Elemente in Verbindungen häufig teilweise oder vollständig gegeneinander ausgetauscht werden. Solche Molybdän-Wolfram-Mischverbindungen weisen in der Regel eine deutlich geringere Wasserlöslichkeit als die jeweiligen Molybdän- bzw. Wolfram-Reinverbindungen auf, ohne dass sich die antimikrobielle Wirksamkeit verschlechtert. Somit wird ein Auswaschen des antimikrobiellen Wirkstoffs besonders zuverlässig verhindert, so dass sich der erfindungsgemäß hergestellte Verbundwerkstoff besonders gut zur Herstellung von Produkten und Gegenständen eignet, die häufig gesäubert werden müssen bzw. in häufigem Kontakt mit Wasser stehen.

In einer weiteren vorteilhaften Ausgestaltung der Erfindung ist vorgesehen, dass die zusätzliche Molybdän- und Wolfram-haltige Mischverbindung mit einer Fluor-Verbindung, insbesondere mit einem Oxyfluorid, WOF₄, WO₂F₂, Calciumfluorid und/oder Fluorapatit, dotiert wird. Auch hierdurch kann die antimikrobielle Wirksamkeit vorteilhaft verbessert und optimal an den jeweiligen Einsatzzweck des Verbundwerkstoffs angepasst werden. Eine Dotierung mit einer Fluor-Verbindung bietet den zusätzlichen Vorteil, dass die Adhäsion von Mikroorganismen an der Oberfläche des Mischoxids zusätzlich erschwert wird. Dies verhindert somit die Besiedelung von mit dem Mischoxid versehenen Oberflächen und verbessert damit den antimikrobiellen Effekt zusätzlich. Vorzugsweise werden hierbei Fluor-Verbindungen mit einer möglichst geringen Wasserlöslichkeit verwendet, um ebenfalls ein Auswaschen zu verhindern oder zumindest zu verlangsamen. Nicht abschließende Beispiele für geeignete Verbindungen sind Calciumfluorid (CaF₂) und Fluorapatit (Ca₅[F|(PO₄)₃]). Die Verwendung von WOF₄, WO₂F₂ und/oder korrespondierenden Molybdänoxyfluoriden bietet den zusätzlichen Vorteil, dass diese gleichzeitig zur Herstellung des Mischoxids und zu dessen Dotierung mit Fluorid-Ionen bzw. Fluor-Verbindungen beitragen.

Alternativ oder zusätzlich ist es vorgesehen, dass als zusätzliche Molybdän- und Wolfram-haltige anorganische Mischverbindung ein Mischoxid der Summenformel MoₓW₁₋ₓA_{y}O_{z} verwendet wird, in welcher 0<x<1, 0≤y≤2 und 2,0≤z≤3,0 betragen und A ein von Mo und W verschiedenes Metall-Ion und/oder NH₄⁺ bezeichnet. Der Parameter x kann somit beispielsweise Werte von 0,01, 0,02, 0,03, 0,04, 0,05, 0,06, 0,07, 0,08, 0,09, 0,10, 0,11, 0,12, 0,13, 0,14, 0,15, 0,16, 0,17, 0,18, 0,19, 0,20, 0,21, 0,22, 0,23, 0,24, 0,25, 0,26, 0,27, 0,28, 0,29, 0,30, 0,31, 0,32, 0,33, 0,34, 0,35, 0,36, 0,37, 0,38, 0,39, 0,40, 0,41, 0,42, 0,43, 0,44, 0,45, 0,46, 0,47, 0,48, 0,49, 0,50, 0,51, 0,52, 0,53, 0,54, 0,55, 0,56, 0,57, 0,58, 0,59, 0,60, 0,61, 0,62, 0,63, 0,64, 0,65, 0,66, 0,67, 0,68, 0,69, 0,70, 0,71, 0,72, 0,73, 0,74, 0,75, 0,76, 0,77, 0,78, 0,79, 0,80, 0,81, 0,82, 0,83, 0,84, 0,85, 0,86, 0,87, 0,88, 0,89, 0,90, 0,91, 0,92, 0,93, 0,94, 0,95, 0,96, 0,97, 0,98 oder 0,99 sowie entsprechende Zwischenwerte annehmen. Der Parameter y kann dementsprechend beispielsweise Werte von 0, 0,01, 0,02, 0,03, 0,04, 0,05, 0,06, 0,07, 0,08, 0,09, 0,10, 0,11, 0,12, 0,13, 0,14, 0,15, 0,16, 0,17, 0,18, 0,19, 0,20, 0,21, 0,22, 0,23, 0,24, 0,25, 0,26, 0,27, 0,28, 0,29, 0,30, 0,31, 0,32, 0,33, 0,34, 0,35, 0,36, 0,37, 0,38, 0,39, 0,40, 0,41, 0,42, 0,43, 0,44, 0,45, 0,46, 0,47, 0,48, 0,49, 0,50, 0,51, 0,52, 0,53, 0,54, 0,55, 0,56, 0,57, 0,58, 0,59, 0,60, 0,61, 0,62, 0,63, 0,64, 0,65, 0,66, 0,67, 0,68, 0,69, 0,70, 0,71, 0,72, 0,73, 0,74, 0,75, 0,76, 0,77, 0,78, 0,79, 0,80, 0,81, 0,82, 0,83, 0,84, 0,85, 0,86, 0,87, 0,88, 0,89, 0,90, 0,91, 0,92, 0,93, 0,94, 0,95, 0,96, 0,97, 0,98, 0,99, 1,00, 1,01, 1,02, 1,03, 1,04, 1,05, 1,06, 1,07, 1,08, 1,09, 1,10, 1,11, 1,12, 1,13, 1,14, 1,15, 1,16, 1,17, 1,18, 1,19, 1,20, 1,21, 1,22, 1,23, 1,24, 1,25, 1,26, 1,27, 1,28, 1,29, 1,30, 1,31, 1,32, 1,33, 1,34, 1,35, 1,36, 1,37, 1,38, 1,39, 1,40, 1,41, 1,42, 1,43, 1,44, 1,45, 1,46, 1,47, 1,48, 1,49, 1,50, 1,51, 1,52, 1,53, 1,54, 1,55, 1,56, 1,57, 1,58, 1,59, 1,60, 1,61, 1,62, 1,63, 1,64, 1,65, 1,66, 1,67, 1,68, 1,69, 1,70, 1,71, 1,72, 1,73, 1,74, 1,75, 1,76, 1,77, 1,78, 1,79, 1,80, 1,81, 1,82, 1,83, 1,84, 1,85, 1,86, 1,87, 1,88, 1,89, 1,90, 1,91, 1,92, 1,93, 1,94, 1,95, 1,96, 1,97, 1,98, 1,99, 2,00 sowie entsprechende Zwischenwerte annehmen. Der Parameter z kann beispielsweise Werte von 2,00, 2,01, 2,02, 2,03, 2,04, 2,05, 2,06, 2,07, 2,08, 2,09, 2,10, 2,11, 2,12, 2,13, 2,14, 2,15, 2,16, 2,17, 2,18, 2,19, 2,20, 2,21, 2,22, 2,23, 2,24, 2,25, 2,26, 2,27, 2,28, 2,29, 2,30, 2,31, 2,32, 2,33, 2,34, 2,35, 2,36, 2,37, 2,38, 2,39, 2,40, 2,41, 2,42, 2,43, 2,44, 2,45, 2,46, 2,47, 2,48, 2,49, 2,50, 2,51, 2,52, 2,53, 2,54, 2,55, 2,56, 2,57, 2,58, 2,59, 2,60, 2,61, 2,62, 2,63, 2,64, 2,65, 2,66, 2,67, 2,68, 2,69, 2,70, 2,71, 2,72, 2,73, 2,74, 2,75, 2,76, 2,77, 2,78, 2,79, 2,80, 2,81, 2,82, 2,83, 2,84, 2,85, 2,86, 2,87, 2,88, 2,89, 2,90, 2,91, 2,92, 2,93, 2,94, 2,95, 2,96, 2,97, 2,98, 2,99 oder 3,00 sowie entsprechende Zwischenwerte annehmen. Hierdurch können die antimikrobiellen, optischen, physikalischen und chemischen Eigenschaften des Verbundwerkstoffs optimal an seine jeweilige Ausgestaltung und seinen jeweiligen Einsatzzweck angepasst werden. Das Mischoxid kann in einfachster Ausgestaltung (y=0) nur Mo, W, O und gegebenenfalls Leerstellen im Kristallgitter enthalten und undotiert sein. Alternativ kann das Mischoxid dotiert sein. Wie bereits erwähnt, kann das Mischoxid neben Mo, W und O auch noch ein oder mehrere von Mo und W verschiedene Metall-Ionen und/oder Ammonium-Ionen innerhalb der durch y definierten Grenzen enthalten. Weiterhin kann vorgesehen sein, dass mehrere unterschiedliche Mischoxide bzw. ein heterogenes Mischoxid mit innerhalb der angegebenen Summenformel variierenden Bestandteilen verwendet werden.

Die wenigstens eine Molybdän-haltige anorganische Verbindung der obigen Formel Aⁿ⁺_{z}MoO₄ wird erfindungsgemäß in Form von Partikeln mit einer mittleren Korngröße zwischen 0,1 µm und 1,00 µm verwendet. Vorteilhafterweise beträgt der Massegehalt der wenigstens einen Molybdän-haltigen Verbindungen der Formel Aⁿ⁺_{z} MoO₄ bezogen auf die Gesamtmasse des Verbundwerkstoffs zwischen 0,1 % und 80 %, insbesondere zwischen 1,5 % und 30 % und vorzugsweise zwischen 1,8 % und 5,0 % beträgt. Hierdurch wird eine besonders hohe antimikrobielle Wirksamkeit bei möglichst geringem Materialeinsatz an Molybdän-haltigen Verbindungen sichergestellt. Zusätzlich kann die Durchführung des Verfahrens optimal an den späteren Einsatzzweck des Verbundwerkstoffs angepasst werden. Die Verwendung von Partikeln mit den genannten mittleren Korngrößen bietet den besonderen Vorteil, dass einerseits eine besonders hohe antimikrobielle Wirksamkeit realisiert werden kann und dass der erfindungsgemäß herstellte Verbundwerkstoff andererseits frei von Nanopartikeln hergestellt werden kann. Unter einer mittleren Korngröße zwischen 0,1 µm und 1,00 µm sind insbesondere mittlere Korngrößen von 0,10 µm, 0,20 µm, 0,30 µm, 0,40 µm, 0,50 µm, 0,60 µm, 0,70 µm, 0,80 µm, 0,90 µm, oder 1,00 µm.

Der Massegehalt der wenigstens einen Molybdän-haltigen Verbindung bezogen auf die Gesamtmasse des Verbundwerkstoffs kann beispielsweise 0,1 %, 0,2 %, 0,3 %, 0,4 %, 0,5 %, 0,6 %, 0,7 %, 0,8 %, 0,9 %, 1,0 %, 1,1 %, 1,2 %, 1,3 %, 1,4 %, 1,5 %, 1,6 %, 1,7 %, 1,8 %, 1,9 %, 2,0 %, 2,1 %, 2,2 %, 2,3 %, 2,4 %, 2,5 %, 2,6 %, 2,7 %, 2,8 %, 2,9 %, 3,0 %, 3,1 %, 3,2 %, 3,3 %, 3,4 %, 3,5 %, 3,6 %, 3,7 %, 3,8 %, 3,9 %, 4,0 %, 4,1 %, 4,2 %, 4,3 %, 4,4 %, 4,5 %, 4,6 %, 4,7 %, 4,8 %, 4,9 %, 5,0 %, 5 %, 6 %, 7 %, 8 %, 9 %, 10 %, 11 %, 12 %, 13 %, 14 %, 15 %, 16 %, 17 %, 18 %, 19 %, 20 %, 21 %, 22 %, 23 %, 24 %, 25 %, 26 %, 27 %, 28 %, 29 %, 30 %, 31 %, 32 %, 33 %, 34 %, 35 %, 36 %, 37 %, 38 %, 39 %, 40 %, 41 %, 42 %, 43 %, 44 %, 45 %, 46 %, 47 %, 48 %, 49 %, 50 %, 51 %, 52 %, 53 %, 54 %, 55 %, 56 %, 57 %, 58 %, 59 %, 60 %, 61 %, 62 %, 63 %, 64 %, 65 %, 66 %, 67 %, 68 %, 69 %, 70 %, 71 %, 72 %, 73 %, 74 %, 75 %, 76 %, 77 %, 78 %, 79 % oder 80 % betragen. Weiterhin kann vorgesehen sein, dass der Masseanteil aller Molybdän-haltigen Verbindungen im Verbundwerkstoff bezogen auf die Gesamtmasse des Verbundwerkstoffs zwischen 0,1 % und 80 % beträgt.

Gemäß der Erfindung wird zusätzlich zu der wenigstens einen Molybdän-haltigen anorganischen Verbindung wenigstens ein Hydrophilierungs- und/oder Hygroskopierungsmittel zumindest im Bereich der Oberfläche des Verbundwerkstoffs angeordnet, wobei das Hydrophilierungs- und/oder Hygroskopierungsmittel Kieselgel oder pyrogenes Siliciumdioxid ist. Hierdurch wird die antimikrobielle Wirksamkeit des erfindungsgemäß hergestellten Verbundwerkstoffs in besonders trockenen Umgebungen, das heißt beispielsweise bei sehr geringer Luftfeuchtigkeit und dementsprechend geringen verfügbaren Wassermengen, die für das Ausbilden einer sauren Oberflächengrenzschicht wichtig sind, vorteilhaft gesteigert. Das Hydrophilierungs- und/oder Hygroskopierungsmittel kann grundsätzlich gemeinsam mit oder getrennt von der wenigstens Molybdän- bzw. Wolfram-haltigen Verbindung verarbeitet werden. Kieselgele und pyrogenes Siliciumdioxid (fumed silica) eignen sich einzeln und in beliebiger Kombination, als Hydrophilierungs- und/oder Hygroskopierungsmittel, da diese eine Art Feuchtigkeitspuffer bilden. Vorzugsweise werden mikronisierte Kieselgele mit einer Partikelgrößenverteilungen im Bereich zwischen 0,1 µm bis 25 µm mittlerer Partikeldurchmesser verwendet. Dadurch kann eine Mindestrestfeuchte im Produkt in weiten Bereichen variiert und eingestellt werden.

Weitere Vorteile ergeben sich, wenn der Massengehalt des obigen Hydrophilierungs- und/oder Hygroskopierungsmittels bezogen auf das Gesamtgewicht des Verbundwerkstoffs zwischen 0,1 % und 15 % beträgt. Beispielsweise kann der Massengehalt 0,1 %, 0,5 %, 1 %, 2 %, 3 %, 4 %, 5 %, 6 %, 7 %, 8 %, 9 %, 10 %, 11 %, 12 %, 13 %, 14 % oder 15 % betragen. Insbesondere kann ein Massengehalt gewählt werden, der zwischen 1 % und 5 %, insbesondere im Bereich von 2 % bis 4 % liegt. Weiterhin kann der Massengehalt bzw. das Massenverhältnis des Hydrophilierungs- und/oder Hygroskopierungsmittels derart gewählt werden, dass er dem gewählten Massengehalt der Molybdän-Verbindung entspricht. Beispielsweise können 2 % des Hydrophilierungs- und/oder Hygroskopierungsmittels verwendet werden, wenn der Massengehalt der Molybdän-haltigen Verbindung bezogen auf das Gewicht des Verbundmaterials ebenfalls 2 % beträgt. Alternativ kann der Massengehalt des Hydrophilierungs- und/oder Hygroskopierungsmittels etwa das Doppelte des Massengehalts der Molybdän-Verbindung betragen. Beispielsweise können 4 % Hydrophilierungs- und/oder Hygroskopierungsmittel verwendet werden, wenn der Massengehalt der Molybdän-haltigen Verbindung 2 % beträgt.

Weiterhin kann vorgesehen sein, dass die wenigstens eine Molybdän-haltige Verbindung zumindest teilweise mit dem Hydrophilierungs- und/oder Hygroskopierungsmittel beschichtet und/oder agglomeriert wird. Hierdurch wird auf einfache Weise eine räumliche Nähe der beiden Verbindungsklassen sichergestellt, so dass die Molybdän-haltige Verbindung auch unter besonders trockenen Bedingungen unmittelbar mit der zur Senkung des pH-Werts erforderlichen Feuchtigkeit versorgt wird.

Ein zweiter Aspekt der Erfindung betrifft einen antimikrobiell wirksamen Verbundwerkstoff wie in Anspruch 1 definiert, wobei erfindungsgemäß vorgesehen ist, dass dieser wenigstens eine Molybdän-haltige anorganische Verbindung umfasst, die mit wenigstens einem weiteren Werkstoff verbunden ist und zusätzlich wenigstens ein Hydrophilierungs- und/oder Hygroskopierungsmittel ausgewählt aus Kieselgel und pyrogenem Siliciumdioxid wenigstens im Bereich der Oberfläche des Verbundwerkstoffs angeordnet ist. Der erfindungsgemäße Verbundwerkstoff ist damit besonders kostengünstig herstellbar und besitzt eine verbesserte Wirksamkeit. Weitere Merkmale und deren Vorteile sind den Beschreibungen des ersten Erfindungsaspekts zu entnehmen, wobei vorteilhafte Ausgestaltungen des ersten Erfindungsaspekts als vorteilhafte Ausgestaltungen des zweiten Erfindungsaspekts und umgekehrt anzusehen sind.

Ein dritter Aspekt der Erfindung betrifft die Verwendung eines antimikrobiell wirksamen Verbundwerkstoffs, welcher mittels eines Verfahrens gemäß dem ersten Erfindungsaspekt erhältlich und/oder erhalten und/oder gemäß dem zweiten Erfindungsaspekt ausgebildet ist, zur Herstellung eines antimikrobiell wirksamen Produkts wie in Anspruch 11 definiert. Das unter Verwendung des antimikrobiell wirksamen Verbundwerkstoffs erhaltene Produkt ist damit besonders kostengünstig herstellbar und besitzt eine verbesserte Wirksamkeit. Weitere Merkmale und deren Vorteile sind den Beschreibungen des ersten und des zweiten Erfindungsaspekts zu entnehmen, wobei vorteilhafte Ausgestaltungen des ersten und des zweiten Erfindungsaspekts als vorteilhafte Ausgestaltungen des dritten Erfindungsaspekts und umgekehrt anzusehen sind.

Weitere Merkmale der Erfindung ergeben sich aus den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht nur in der jeweils angegebenen Kombination, sondern auch in anderen Kombinationen verwendbar, ohne den Rahmen der Erfindung zu verlassen. Dabei zeigt:
- Fig. 1: eine schematische Schnittansicht eines antimikrobiell wirksamen Verbundwerkstoffs, welcher im Bereich seiner Oberfläche eine Molybdän-haltige anorganische Verbindung umfasst; und
- Fig. 2: eine schematische Schnittansicht eines weiteren antimikrobiell wirksamen Verbundwerkstoffs, welcher eine Wolfram-haltige anorganische Verbindung umfasst.

In einem ersten Ausführungsbeispiel werden die Molybdate CaMO₄, ZnMO₄, BiMO₄, VMO₄, CuMO₄ und Ag₂MO₄ mit M=Mo, und Vergleichsbeispiele mit M=W hergestellt, indem MoO₃ und/oder WO₃ mit den entsprechenden Carbonaten, beispielsweise CaCO₃, MgCO₃, ZnCO₃, BiCO₃ etc. in äquimolaren Verhältnissen innig vermischt und das Gemisch auf Temperaturen von etwa 400 °C bis 800 °C erhitzt wird. Dabei können grundsätzlich auch unterschiedliche Carbonate verwendet werden, um entsprechende Mischmolybdate bzw. Mischwolframate zu erhalten. Die Reaktion kann durch Entfernen des entstehenden CO₂ in Richtung der gewünschten Produkte getrieben werden. Sobald kein Carbonat mehr nachweisbar ist, ist die Umsetzung vollständig erfolgt. Alternativ können verschiedene Molybdate/Wolframate durch Zutropfen einer Metallsalz-Lösung - z. B. einer AgNO₃-Lösung - zu einer Na₂MoO₄- bzw. Na₂WO₄-Lösung mit anschließendem Abtrennen des gebildeten Molybdat/Wolframat-Niederschlags hergestellt werden. Der Niederschlag kann dann bedarfsweise gewaschen und getrocknet werden. Diese Herstellungsvariante bietet sich in Fällen an, in denen das gebildete Molybdat/Wolframat eine geringe Wasserlöslichkeit als Na₂MoO₄ bzw. Na₂WO₄ und als das verwendete Metallsalz besitzt. Die genannten Verbindungen zeigen neben einer guten antimikrobiellen Wirkung auch eine besonders hohe Licht- und UV-Stabilität auf. Die Prüfung der UV-Beständigkeit kann in Anlehnung an DIN EN 438-2, Abschnitt 27 durchgeführt werden. Hierbei wird eine Probe eines Verbundwerkstoffs hergestellt, indem eine oder mehrere der genannten Molybdate bzw. Wolframate mit einem weiteren Werkstoff verbunden werden. Die Molybdate bzw. Wolframate können dabei als Schicht bzw. Bestandteil einer Schicht vorliegen und/oder im wenigstens einen weiteren Werkstoff verteilt vorliegen. Der Verbundwerkstoff wird für 60 Minuten einer Bestrahlung ausgesetzt. Gleiches erfolgt mit einer analog, aber ohne Zusatz der Molybdän-/Wolfram-Verbindungen hergestellten Vergleichsprobe ("Standard-Produkt"). Die Auswertung erfolgt dann anhand eines visuellen Vergleichs der antimikrobiell ausgerüsteten und der nicht ausgerüsteten Probe und wird beispielsweise wie folgt bewertet:
1: kein wahrnehmbarer Unterschied zum Standard-Produkt
2: schwer wahrnehmbarer Unterschied zum Standard-Produkt
3: eindeutig wahrnehmbarer Unterschied zum Standard-Produkt
4: gerade noch akzeptabler Unterschied zum Standard-Produkt
5: nicht akzeptabler Unterschied zum Standard-Produkt

Für die genannten Molybdate/Wolframate werden dabei stets Werte von 1 oder höchstens 2 erhalten.

Gleiche Versuche wurden als Vergleichsbeispiele mit Verbindungen der allgemeinen Formel MO₃₋ₓ mit 0<x<1 und M=W, Mo durchgeführt. Hierzu wurden MoO₂ und WO₂, einzeln oder in bestimmten Mischungsverhältnissen, partiell oxidiert bzw. MoO₃ und/oder WO₃ partiell reduziert. Die resultierenden Oxide bzw. Mischoxide wiesen ebenfalls hervorragende antimikrobielle Eigenschaften auf, wenn sie als Verbundwerkstoff mit wenigstens einem weiteren Werkstoff vorlagen. Beispiele hierfür sind MoO_{2,35} bis MoO_{2,97} und WO_{2,35} bis WO_{2,97}.

Als weitere Werkstoffe kommen grundsätzlich beispielsweise Kunststoffe, Farben, Lacke, Silikone, Gummi, Kautschuk, Melamin, Acrylate, Methylacrylate, Wachse, Epoxydharze, Glas, Metall, Keramik und weitere in Frage. Der Werkstoff, in welchen zum Zwecke der antimikrobiellen Ausstattung das oder die Molybdän-Verbindung(en) eingebracht wird bzw. werden, kann eine feste und/oder flüssige Matrix bilden. Es kann vorgesehen sein, dass die Molybdän-Verbindungen derart zugegeben werden, dass sie zwischen 0,1 % und 10 % (Gewichts- oder Volumenprozente) des Gesamtgewichts bzw. Gesamtvolumens ausmachen. Weiterhin kann vorgesehen sein, dass die Molybdän-Verbindungen in partikulärer Form mit mittleren Teilchengrößen zwischen 0,1 µm und 100 µm verwendet werden.

Beispielsweise kann der wenigstens eine weitere Werkstoff hydrophobe Polymere wie Silikone, Polypropylen (PP), Acrylnitril-Butadien-Styrol (ABS), Polycarbonat (PC) oder Polystyrol (PS) umfassen bzw. aus diesen bestehen. Ebenso vorgesehen sein können Phenol-Harze, Phenolformaldehyd-Harze, Melamin-Harze, Melaminformaldehyd-Harze, Harnstoff-Harze, Harnstoffformaldehyd-Harze und polymeres Diphenylmethandiisocyanat sowie beliebige Mischungen daraus. Weiterhin kann der Verbundwerkstoff als weiteren Werkstoff Polyethylen (PE), Polyethylenterephthalat (PET), Polyvinylchlorid (PVC), Polystyrol (PS), Polycarbonat (PC) oder ein Poly(meth)acrylat (z. B. PAA, PAN, PMA, PBA, ANBA, ANMA, PMMA, AMMA, MABS und/oder MBS) umfassen. Die Verwendung von thermoplastischen Elastomeren erlaubt die Herstellung von Oberflächen mit gummielastischen Eigenschaften, in welchen die wenigstens eine Molybdän-/Wolfram-haltige Verbindung aufgenommen bzw. gehalten ist. Das oder die thermoplastischen Elastomere können beispielsweise den Klassen TPO, TPV, TPU, TPC, TPS oder TPA bzw. beliebigen Mischungen hieraus angehören, wobei sich insbesondere thermoplastische Elastomere auf Urethanbasis (TPUs) als vorteilhaft gezeigt haben. Die Verwendung eines Reaktivlacks erlaubt die Herstellung von mechanisch besonders widerstandsfähigen Oberflächen, wobei der Reaktivlack durch chemische Reaktion vorzugsweise bereits bei Raumtemperatur erhärtet. Der Reaktivlack kann grundsätzlich als Ein- oder Mehrkomponentenlack vorliegen bzw. verwendet werden. Ebenso kann der Verbundwerkstoff grundsätzlich als UV-härtbarer Lack, Acryllack und/oder Silikon-haltiger Lack ausgebildet sein. Im Fall der Ausgestaltung als UV-härtbarer Lack hat sich die Verwendung von Licht- und UV-stabilen Molybdän-/Wolfram-haltigen Verbindungen als vorteilhaft gezeigt, um Verfärbungen zu vermeiden. Umgekehrt können aber auch Licht- bzw. UV-labile Molybdän-haltige Verbindungen verwendet und gleichzeitig mit der Aushärtung des Lacks umgewandelt werden. Verbundwerkstoff-Lacke auf Silikon-Basis besitzen aufgrund ihres geringen Anteils an organischen Gruppen den Vorteil einer sehr geringen Änderung ihres Filmvolumens während der Härtung. Hierdurch können sehr dichte Schichten mit guter Filmfestigkeit erzeugt werden, in denen die wenigstens eine Molybdän-haltige Verbindung aufgenommen bzw. gehalten ist. Darüber hinaus weisen Silikon-Lacke eine hohe thermische Beständigkeit auf und eignen sich daher zur Beschichtung von Gegenständen, die zur Verwendung im Bereich von Wärmequellen vorgesehen sind.

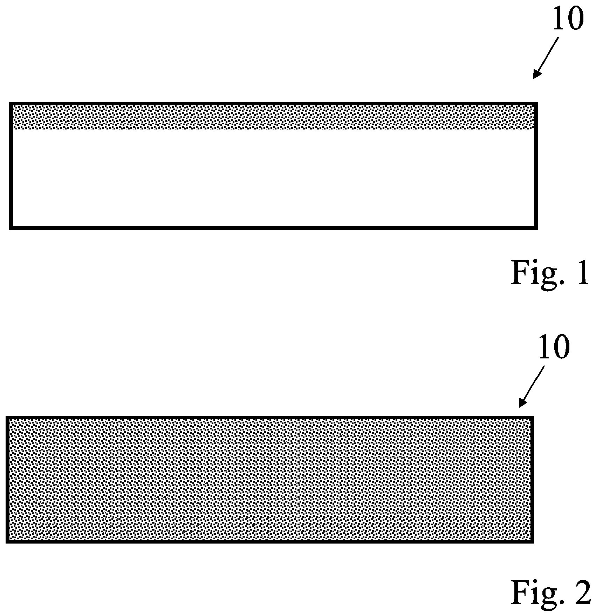

Fig. 1 zeigt zur weiteren Verdeutlichung eine schematische Schnittansicht eines antimikrobiell wirksamen Verbundwerkstoffs 10, welcher nur im Bereich seiner Oberfläche ZnMoO₄ als Molybdän-haltige anorganische Verbindung umfasst. Das ZnMoO₄, welches gepunktet dargestellt ist, liegt dabei mit einem Massenanteil von 2 % in Polypropylen (PP) vor. Der ZnMoO₄-haltige Oberflächenbereich bildet vorzugsweise eine Außenseite eines aus dem Verbundwerkstoff 10 hergestellten Produkts.

Fig. 2 zeigt eine schematische Schnittansicht eines weiteren antimikrobiell wirksamen Verbundwerkstoffs 10, als Vergleichsbeispiel, welcher WO_{2,97} als Wolfram-haltige anorganische Verbindung 12 umfasst. Man erkennt, dass das Wolframoxid im Unterschied zum vorhergehenden Beispiel nicht nur an der Oberfläche, sondern fein verteilt im gesamten Volumen des Verbundwerkstoffs 10 vorliegt, wobei als weiterer Werkstoff erneut eine PP-Matrix verwendet wurde.

Als weitere Ausführungsbeispiele und Vergleichsbeispiele (§) wurden die folgenden Verbundwerkstoffe hergestellt, die alle eine hervorragende antimikrobielle Wirksamkeit zeigten:
PP + 2% WO₃₋ₓ (blau) + 2% Silica Gel (§)
PP + 2% WO₃ (gelb) + 2% Silica Gel (§)
PP + 2% MoO₃ + 2% Silica Gel + 2% Atmer (§)
PP + 2% ZnMoO₄ + 2% Silica Gel+ 2% Atmer
PP + 4% ZnMoO₄ (§)
PP + 4% MoO₃ (§)
PP + 4% WO₃₋ₓ (blau) (§)
PP + 4% WO₃₋ₓ (blau) + 2% Silica Gel (§)
PP + 4% WO₃ (gelb) + 2% Silica Gel (§)
PP + 4% WO₃ (gelb) + 2% Silica Gel (§)

Silica Gel und Atmer fungieren dabei als Hydrophilierungs- bzw. Hygroskopierungsmittel.

Zur Herstellung wurden die Molybdän- bzw. Wolfram-Verbindungen sowie gegebenenfalls die Hydrophilierungs- bzw. Hygroskopierungsmittel in pulverförmiger Form mit mittleren Partikeldurchmessern zwischen 0,1 µm und 5 µm in einem Brabender Plasticorder in das PP eingemischt. Es ist zu betonen, dass grundsätzlich auch andere Kunststoffe wie beispielsweise PE, TPU, PU und dergleichen verwendet werden können. Weiterhin können neben Druckguss- auch Spritzgussmaterialien antimikrobiell ausgestattet werden. Gleiches gilt für Verbundwerkstoffe, die neben Molybdän-/Wolfram-haltigen Verbindungen anorganische Werkstoffe als Matrix umfassen.

Die einzelnen Proben wurden etwa 3 Stunden vor der ersten Testung der antimikrobiellen Wirksamkeit mit Wasser besprüht, um eine ausreichende Restfeuchtigkeit im Verbundwerkstoff 10 sicherzustellen. Die Testung auf antimikrobielle Wirksamkeit erfolgte nach dem Abtrocknen auf trockener Oberfläche. Hierzu wurden die einzelnen, abgetrockneten Verbundwerkstoffe zunächst mit einer Keimsuspension (Keimzahl 10⁶) kontaminiert. Als Keime wurden Staphylococcus aureus (s.a.), Pseudomonas aeruginosa (p.a.) und Escherichia coli (E. coli) verwendet. Die jeweilige Konzentration der Keimsuspension wurde über photometrische Messung ermittelt und eingestellt.

Je 10 µL der Keimsuspension wurde mit Hilfe einer Pipette auf das Verbundmaterial 10 aufgetragen und mit einem Drigalskispatel ausgestrichen. Nach der vollständigen Eintrocknung der Keimsuspension (nach etwa einer Stunde ohne weitere Berührung oder Behandlung) wurden sogenannte Abklatschtests nach 0, 3, 6 und 9 Stunden durchgeführt. Hierzu wird eine Caso-Agar Kontaktplatte auf den kontaminierten Oberflächenbereich gedrückt und danach 24 Stunden in an sich bekannter Weise bebrütet. Bei den Verbundwerkstoffen 10 konnte in der Regel nach 3 Stunden, aber spätestens nach 6 Stunden kein Keimwachstum mehr festgestellt werden. Entsprechende Ergebnisse konnten auch mit der an sich bekannten Auftropfmethode erzielt werden.

Die antimikrobielle Wirkung konnte in sehr trockenen Umgebungen mit Luftfeuchtigkeitswerten unter 20 % auch bei denjenigen Verbundwerkstoffen 10 ohne Hydrophilierungs- bzw. Hygroskopierungsmittel über mehrere Wochen aufrechterhalten werden. Die Verwendung von Hydrophilierungs- bzw. Hygroskopierungsmittel verbessert und verlängert aber insbesondere unter sehr trockenen Bedingungen die antimikrobielle Wirksamkeit. Bei sehr niedrigen Luftfeuchtigkeitswerten kann die antimikrobielle Wirksamkeit nach einigen Tagen durch erneutes Anfeuchten, beispielsweise durch Abwischen der Oberfläche mit einem feuchten Tuch, wieder regeneriert werden. Bei höheren Luftfeuchtigkeiten über etwa 30 % sind keine besonderen Maßnahmen erforderlich, um die antimikrobielle Wirksamkeit dauerhaft aufrecht zu erhalten.

Der Verbundwerkstoff 10 kann zur Herstellung unterschiedlichster Produkte und Gegenstände verwendet werden. Das Produkt kann beispielsweise als Implantat, Katheter, Stent, Knochenimplantat, Zahnimplantat, Gefäßprothese, Endoprothese, Exoprothese, Kabel, Schlauch, Lebensmittelverpackung, Behälter, Kraftstofftank, Haushaltsprodukt, Schalter, Armatur, Tastatur, Maus, Joystick, Gehäuse, Textil, Bekleidungsstück, Möbel- und/oder Innenausbauteil, Haushaltsgerät, Kreditkarte, Handygehäuse, Münze, Geldschein, Türschnalle, Kühlschrank, Rieselkörper im Kühlturm, Lackbeschichtung, Fliese oder ein Teil der Innenausstattung eines Gebäudes oder öffentlichen Verkehrsmittels ausgebildet sein. Weiterhin kann vorgesehen sein, dass das Produkt als Lager- und Transportbehälter oder als Leitung für Kohlenwasserstoffe, Treibstoffe, Lösungsmittel und organische Flüssigkeiten ausgebildet ist. Ebenso kann der erfindungsgemäße Verbundwerkstoff zur Herstellung eines Gegenstands aus der Gruppe der Haushaltswaren, Medizintechnik, Lebensmitteltechnik, Sanitäranlagen, Verpackungen, Textilien, Klinken, Schalter, Sitze und Tastaturen verwendet werden. Ebenso eignet er sich für Produkte, die im häufigen Berührungskontakt mit Lebewesen stehen. Eine weitere vorteilhafte Verwendung liegt in Bauteilen für Klimaanlagen. Die Kühlrippen, die üblicherweise aus einer Cu- oder AI-Legierung bestehen, können in vorteilhafter Weise mit dem erfindungsgemäßen Verbundwerkstoff beschichtet oder aus diesem hergestellt werden. Auch die Schächte von Klimaanlagen in Gebäuden können antimikrobiell ausgeführt werden, indem der Verbundwerkstoff 10 dem Schachtmaterial zugesetzt wird, das Schachtmaterial mit diesen beschichtet wird oder wenn das Schachtmaterial aus dem Verbundwerkstoff 10 besteht. Auch Luftbefeuchter können mit entsprechenden antimikrobiellen Eigenschaften versehen werden. Außerdem kann der Verbundwerkstoff in Kabeln und/oder zur Herstellung von Kabeln verwendet werden.

In weiterer Ausgestaltung der Erfindung ist der Verbundwerkstoff als Beschichtungsmittel, insbesondere als Anstrichmittel, Lack und/oder Antifouling-Anstrich, ausgebildet. Unter einem Anstrichmittel werden Ausführungsformen des Verbundwerkstoffs verstanden, die eine flüssige bis pastenförmige Konsistenz besitzen und die auf Oberflächen aufgetragen einen physikalisch oder chemisch trocknenden Anstrich ergeben. Hierdurch können die vorteilhaften Eigenschaften des erfindungsgemäßen Verbundwerkstoffs besonders flexibel für beliebige Gegenstände und Oberflächen verwirklicht werden. Wichtige Ausgestaltungen sind beispielsweise AntiFouling Anstriche, z. B. für Schiffe, sowie antimikrobielle Ausstattung im Gesundheitswesen, der Industrie, dem Lebensmittelbereich und dem privaten Sektor.

In einem weiteren Ausführungsbeispiel wird zumindest die Oberfläche des Verbundwerkstoffs hydrophilisiert.

Der Massegehalt des Hydrophilierungs- und/oder Hygroskopierungsmittels kann bezogen auf die Masse des Verbundwerkstoffs zwischen 0,1 % und 10 %, insbesondere zwischen 0,15 % und 5 % und vorzugsweise zwischen 0,2 % und 4 % betragen. Als Hydrophilierungs- und/oder Hygroskopierungsmittel kann beispielsweise Kieselgel mit Massegehalten von etwa 2 % verwendet werden, wodurch sich ein Gesamtmassegehalt von 4 % Hydrophilierungs- bzw. Hygroskopierungsmittel ergibt. Alternativ können beispielsweise auch nur 2 % Kieselgel verwendet werden.

Mit Hilfe des Hydrophilierungs- und/oder Hygroskopierungsmittels kann der Verbundwerkstoff auch in besonders trockenen Umgebungen mit Luftfeuchtigkeitswerten unter 20 % verwendet werden. Zum Aktivieren bzw. Regenerieren der antimikrobiellen Wirksamkeit ist es dabei ausreichend, die Oberfläche des Verbundwerkstoffs beispielsweise im Wochenrhythmus anzufeuchten, beispielsweise mit einem feuchten Tuch abzuwischen.

Unter hygroskopisch ist zu verstehen, dass das Verbundmaterial zumindest an seiner Oberfläche bzw. in den oberflächennahen Bereichen Feuchtigkeit aufnimmt. Beispielsweise sollte das Verbundmaterial in Umgebungen mit <10% relativer Luftfeuchtigkeit zwischen 0,01 bis 10 Gew.-% Feuchtigkeit aufnehmen. Besonders vorteilhaft sind 0,1 bis 3% Gleichgewichtsfeuchtigkeit, die sich in der Regel nach einigen Minuten bis Stunden einstellen.

## Patentansprüche

1. Verfahren zum Herstellen eines antimikrobiell wirksamen Verbundwerkstoffs (10), bei welchem wenigstens eine Molybdän-haltige anorganische Verbindung mit wenigstens einem weiteren Werkstoff verbunden wird, **dadurch gekennzeichnet, dass**
als anorganische Molybdän-haltige Verbindung wenigstens eine Verbindung der Summenformel Aⁿ⁺_{z}MO₄ verwendet wird, in welcher M Mo bedeutet, A ein von Mo verschiedenes Metall-Ion aus einer Gruppe, die aus Na, K, Mg, Ca, Ag, Cu, Bi, V, Ti und Zn besteht, bezeichnet und n*z=+2 beträgt, wobei die wenigstens eine Molybdän-haltige anorganische Verbindung der Summenformel Aⁿ⁺_{z}MO₄ in Form von Partikeln mit einer mittleren Korngröße zwischen 0,1 µm und 1,00 µm verwendet wird und
**dadurch gekennzeichnet, dass**
zusätzlich zu der wenigstens einen Molybdän-haltigen anorganischen Verbindung wenigstens ein Hydrophilierungs- und/oder Hygroskopierungsmittel zumindest im Bereich der Oberfläche des Verbundwerkstoffs (10) angeordnet wird, wobei das Hydrophilierungs- und/oder Hygroskopierungsmittel Kieselgel oder pyrogenes Siliciumdioxid umfasst.

2. Verfahren nach Anspruch 1,
**dadurch gekennzeichnet, dass**
wenigstens eine Molybdän-haltige anorganische Verbindung verwendet wird, welche dotiert und/oder undotiert und/oder kristallwasserfrei und/oder kristallwasserhaltig ist.

3. Verfahren nach Anspruch 1 oder 2,
**dadurch gekennzeichnet, dass**
als weitere anorganische Verbindung MO₃₋ₓ mit M=Mo und/oder W und 0<x<1 verwendet wird.

4. Verfahren nach Anspruch 3,
**dadurch gekennzeichnet, dass**
die Verbindung MO₃₋ₓ durch partielle Oxidation von M und/oder MO₂ und/oder durch partielle Reduktion von MO₃ hergestellt wird.

5. Verfahren nach Anspruch 3 oder 4,
**dadurch gekennzeichnet, dass**
die Verbindung MO₃₋ₓ mittels eines Wirbelschichtreaktors und/oder durch chemische Gasphasenabscheidung und/oder durch physikalische Gasphasenabscheidung und/oder durch Sputtern und/oder plasmaassistiert und/oder in einer kontrollierten Atmosphäre hergestellt wird.

6. Verfahren nach einem der Ansprüche 1 bis 5,
**dadurch gekennzeichnet, dass**
zusätzlich ein Mischoxid der Summenformel MoₓW₁₋ₓA_{y}O_{z} verwendet wird, in welcher 0<x<1, 0≤y≤2 und 2,0≤z≤3,0 betragen und A ein von Mo und W verschiedenes Metall-Ion und/oder NH₄⁺ bezeichnet.

7. Verfahren nach Anspruch 6,
**dadurch gekennzeichnet, dass**
A ausgewählt wird aus einer Gruppe, die Na, K, Mg, Ca, Ag, Cu, Bi, V, Ti und Zn umfasst und/oder dass das Mischoxid mit einer Fluor-Verbindung, insbesondere mit einem Oxyfluorid, WOF₄, WO₂F₂, Calciumfluorid und/oder Fluorapatit, dotiert wird.

8. Verfahren nach einem der Ansprüche 1 bis 7,
**dadurch gekennzeichnet, dass**
der Massegehalt der wenigstens einen Molybdän-haltigen Verbindung bezogen auf die Gesamtmasse des Verbundwerkstoffs (10) zwischen 0,1 % und 80 %, insbesondere zwischen 1,5 % und 30 % und vorzugsweise zwischen 1,8 % und 5,0 % beträgt.

9. Verfahren nach einem der vorhergehenden Ansprüche,
**dadurch gekennzeichnet, dass**
die wenigstens eine Molybdän-haltige anorganische Verbindung zumindest teilweise mit dem Hydrophilierungs- und/oder Hygroskopierungsmittel beschichtet und/oder agglomeriert wird.

10. Antimikrobiell wirksamer Verbundwerkstoff (10), welcher wenigstens eine Molybdän-haltige anorganische Verbindung umfasst, die mit wenigstens einem weiteren Werkstoff verbunden ist,
**dadurch gekennzeichnet, dass**
die anorganische Molybdän-haltige Verbindung wenigstens eine Verbindung der Summenformel Aⁿ⁺_{z}MO₄ ist, in welcher M Mo bedeutet, A ein von Mo verschiedenes Metall-Ion aus einer Gruppe, die aus Na, K, Mg, Ca, Ag, Cu, Bi, V, Ti und Zn besteht, bezeichnet und n*z=+2 beträgt, wobei die wenigstens eine Molybdän-haltige anorganische Verbindung der Summenformel Aⁿ⁺_{z}MO₄ in Form von Partikeln mit einer mittleren Korngröße zwischen 0,1 µm und 1,00 µm vorliegt und
**dadurch gekennzeichnet, dass**
zusätzlich zu der wenigstens einen Molybdän-haltigen anorganischen Verbindung wenigstens ein Hydrophilierungs- und/oder Hygroskopierungsmittel zumindest im Bereich der Oberfläche des Verbundwerkstoffs (10) angeordnet wird, wobei das Hydrophilierungs- und/oder Hygroskopierungsmittel Kieselgel oder pyrogenes Siliciumdioxid umfasst.

11. Verwendung eines antimikrobiell wirksamen Verbundwerkstoffs (10), welcher mittels eines Verfahrens nach einem der Ansprüche 1 bis 9 erhältlich und/oder erhalten und/oder gemäß Anspruch 10 ausgebildet ist, zur Herstellung eines antimikrobiell wirksamen Produkts.

## Claims

1. Method for preparing an antimicrobially active composite material (10), in which method at least one molybdenum-containing inorganic compound is combined with at least one further material, **characterised in that** at least one compound of total formula Aⁿ⁺_{Z}MO₄ is used as the inorganic molybdenum-containing compound, in which formula M represents Mo, A denotes a metal ion other than Mo from a group consisting of Na, K, Mg, Ca, Ag, Cu, Bi, V, Ti and Zn, and n*z=+2, the at least one molybdenum-containing inorganic compound of total formula Aⁿ⁺_{Z}MO₄ being used in the form of particles having an average particle size between 0.1 µm and 1.00 µm and **characterised in that**, in addition to the at least one molybdenum-containing inorganic compound, at least one hydrophilising agent and/or hygroscopic agent is arranged at least in the region of the surface of the composite material (10), the hydrophilising agent and/or hygroscopic agent comprising silica gel or pyrogenic silicon dioxide.

2. Method according to claim 1, **characterised in that** at least one molybdenum-containing inorganic compound is used which is doped and/or undoped and/or free from water of crystallization and/or contains water of crystallization.

3. Method according to claim 1 or 2, **characterised in that** MO_{3-X} is used as the further inorganic compound, where M=Mo and/or W and 0<x<1.

4. Method according to claim 3, **characterised in that** the compound MO_{3-X} is prepared by partial oxidation of M and/or MO₂ and/or by partial reduction of MO₃.

5. Method according to claim 3 or 4, **characterized in that** the compound MO_{3-X} is prepared by means of a fluidized bed reactor and/or by chemical vapour deposition and/or by physical vapour deposition and/or by sputtering and/or by plasma assistance and/or in a controlled atmosphere.

6. Method according to any of claims 1 to 5, **characterised in that** a mixed oxide of total formula MOₓW_{1-X}A_{y}O_{z} is additionally used, in which formula 0<x<1, 0<y<2 and 2.0<z<3.0 and A denotes a metal ion other than Mo and W and/or denotes NH₄⁺.

7. Method according to claim 6, **characterised in that** A is selected from a group comprising Na, K, Mg, Ca, Ag, Cu, Bi, V, Ti and Zn and/or **in that** the mixed oxide is doped with a fluorine compound, in particular with an oxyfluoride, WOF₄, WO₂F₂, calcium fluoride and/or fluoroapatite.

8. Method according to any of claims 1 to 7, **characterised in that** the mass content of the at least one molybdenum-containing compound, based on the total mass of the composite material (10), is between 0.1% and 80%, in particular between 1.5% and 30%, and preferably between 1.8% and 5.0%.

9. Method according to any of the preceding claims, **characterised in that** the at least one molybdenum-containing inorganic compound is at least partially coated and/or agglomerated with the hydrophilising agent and/or hygroscopic agent.

10. Antimicrobially active composite material (10) comprising at least one molybdenum-containing inorganic compound that is combined with at least one further material, **characterised in that** the inorganic molybdenum-containing compound is at least one compound of total formula Aⁿ⁺_{Z}MO₄ in which M represents Mo, A denotes metal ion other than Mo from a group consisting of Na, K, Mg, Ca, Ag, Cu, Bi, V, Ti and Zn, and n*z=+2, the at least one molybdenum-containing inorganic compound of total formula Aⁿ⁺_{Z}MO₄ being present in the form of particles having an average particle size between 0.1 µm and 1.00 µm and **characterised in that**, in addition to the at least one molybdenum-containing inorganic compound, at least one hydrophilising agent and/or hygroscopic agent is arranged at least in the region of the surface of the composite material (10), the hydrophilising and/or hygroscopic agent comprising silica gel or pyrogenic silicon dioxide.

11. Use of an antimicrobially active composite material (10) which can be obtained and/or is obtained by means of a method according to any of claims 1 to 9 and/or is designed according to claim 10, for preparing an antimicrobially active product.

## Revendications

1. Procédé pour fabriquer un matériau composite efficace du point de vue antimicrobien (10), dans lequel au moins un composé inorganique contenant du molybdène est combiné avec au moins un autre matériau,
**caractérisé en ce que**
comme composé inorganique contenant du molybdène est utilisé au moins un composé de formule brute Aⁿ⁺_{z}MO₄ dans laquelle M signifie Mo, A désigne un ion métallique différent de Mo d'un groupe qui consiste en Na, K, Mg, Ca, Ag, Cu, Bi, V, Ti et Zn, et n^{∗}z = +2, où le au moins un composé inorganique contenant du molybdène de formule brute Aⁿ⁺_{z}MO₄ est utilisé sous forme de particules ayant une taille de grains moyenne entre 0,1 µm et 1,00 µm et
**caractérisé en ce que**
en plus du au moins un composé inorganique contenant du molybdène au moins un agent d'hydrophilie et/ou d'hygroscopie est disposé au moins dans le domaine de la surface du matériau composite (10), où l'agent d'hydrophilie et/ou d'hygroscopie comprend du gel de silice ou du dioxyde de silicium pyrogène.

2. Procédé selon la revendication 1,
**caractérisé en ce que**
au moins un composé inorganique contenant du molybdène qui est dopé et/ou non dopé et/ou sans eau de cristallisation et/ou contenant de l'eau de cristallisation est utilisé.

3. Procédé selon la revendication 1 ou 2,
**caractérisé en ce que**
MO₃₋ₓ avec M = Mo et/ou W et 0 < x < 1 est utilisé comme autre composé inorganique.

4. Procédé selon la revendication 3,
**caractérisé en ce que**
le composé MO₃₋ₓ est fabriqué par oxydation partielle de M et/ou MO₂ et/ou par réduction partielle de MO₃.

5. Procédé selon la revendication 3 ou 4,
**caractérisé en ce que**
le composé MO₃₋ₓ est fabriqué au moyen d'un réacteur à couche fluidisée et/ou par dépôt chimique en phase gazeuse et/ou par dépôt physique en phase gazeuse et/ou par pulvérisation cathodique et/ou de manière assistée par plasma et/ou dans une atmosphère contrôlée.

6. Procédé selon l'une des revendications 1 à 5,
**caractérisé en ce que**
en outre un oxyde mixte de formule brute MoₓW₁₋ₓA_{y}O_{z} est utilisé, dans lequel 0 < x < 1, 0 ≤ y ≤ 2 et 2,0 ≤ z ≤ 3,0 et A désigne un ion métallique différent de Mo et W et/ou NH₄⁺.

7. Procédé selon la revendication 6,
**caractérisé en ce que**
A est choisi dans un groupe qui comprend Na, K, Mg, Ca, Ag, Cu, Bi, V, Ti et Zn et/ou **en ce que** l'oxyde mixte est dopé avec un composé du fluor, en particulier avec un oxyfluorure, WOF₄, WO₂F₂, le fluorure de calcium et/ou la fluoroapatite.

8. Procédé selon l'une des revendications 1 à 7,
**caractérisé en ce que**
la teneur en masse du au moins un composé contenant du molybdène par rapport à la masse totale du matériau composite (10) est entre 0,1 % et 80 %, en particulier entre 1,5 % et 30 % et de préférence entre 1,8 % et 5,0 %.

9. Procédé selon l'une des revendications précédentes,
**caractérisé en ce que**
le au moins un composé inorganique contenant du molybdène est revêtu et/ou aggloméré au moins en partie avec l'agent d'hydrophile et/ou d'hygroscopie.

10. Matériau composite efficace du point de vue antimicrobien (10), qui comprend au moins un composé inorganique contenant du molybdène qui est combiné avec au moins un autre matériau,
**caractérisé en ce que**
le composé inorganique contenant du molybdène est au moins un composé de formule brute Aⁿ⁺_{z}MO₄ dans laquelle M signifie Mo, A désigne un ion métallique différent de Mo d'un groupe qui consiste en Na, K, Mg, Ca, Ag, Cu, Bi, V, Ti et Zn, et n^{∗}z = +2, où le au moins un composé inorganique contenant du molybdène de formule brute Aⁿ⁺_{z}MO₄ est utilisé sous forme de particules ayant une taille de grains moyenne entre 0,1 µm et 1,00 µm et
**caractérisé en ce que**
en plus du au moins un composé inorganique contenant du molybdène au moins un agent d'hydrophilie et/ou d'hygroscopie est disposé au moins dans le domaine de la surface du matériau composite (10), où l'agent d'hydrophilie et/ou d'hygroscopie comprend du gel de silice ou du dioxyde de silicium pyrogène.

11. Utilisation d'un matériau composite efficace du point de vue antimicrobien (10), qui peut être obtenu et/ou est obtenu au moyen d'un procédé selon l'une des revendications 1 à 9 et/ou est formé selon la revendication 10, pour la fabrication d'un produit efficace du point de vue antimicrobien.
